# EUROPEAN PATENT APPLICATION

(11) **EP 1 953 236 A1**
(43) Date of publication of application: **06.08.2008**
(21) Application number: 07251763.4
(22) Date of filing: 26.04.2007
(51) Int. Cl.: C12P 19/26

(54) **Methods for producing glucosamine from microbial biomass**

(30) Priority: 01.02.2007 US 701111
(71) Applicant: Hygieia Health Co., Ltd, Central Hong Kong (HK)
(72) Inventor: Cao, Liqun, Xuzhou, Jiangsu Province (CN); Yu, Yuanming, Xuzhou, Jiangsu Province (CN); Li, Wuzhou, Xuzhou, Jiangsu Province (CN); Jiang, Yonghong, Xuzhou, Jiangsu Province (CN); Wei, Xianzhong, Xuzhou, Jiangsu Province (CN)
(74) Representative: Stuttard, Garry Philip

(57) **Abstract**

A more efficient method for producing glucosamine from microbial biomass such as, for example, fungal biomass is disclosed. In one embodiment, a method for obtaining glucosamine from fungal biomass includes providing fungal biomass containing chitin, such as Aspergillus, Penicillium, Mucor, or mushroom. The fungal biomass is then reacted in an acidic solution with an acid concentration of greater than about 20% by weight and at a reaction temperature greater than about 60° C to convert the chitin to glucosamine. This reaction is performed for a period of less than 4 hours. The glucosamine is then separated from the acidic solution. In selected embodiments, dried fungal biomass is used to accelerate the reaction. This dried fungal biomass may, in some embodiments, have a moisture content of less than about 20% and in some cases less than about 13%.

## Description

This invention relates to glucosamine production and, more particularly, to more efficient methods for producing glucosamine from microbial biomass such as, for example, fungal biomass.

Glucosamine is a molecule containing glucose and an amine which is produced naturally in the body to promote healthy cartilage. It is believed that glucosamine molecules stimulate cartilage cells to generate two proteins, proteoglycans and collagen, that aid in holding joint tissue together. Glucosamine is not typically obtained directly from food, but is generated by the body using building blocks that are found in food.

As the body ages, it loses its ability to produce glucosamine. This reduces cartilage flexibility and renders it more susceptible to injury. Furthermore, individuals with arthritis typically have a faster rate of cartilage breakdown, and a slower rate of cartilage production. To offset these effects, glucosamine is widely taken as a supplement in an effort to provide more cartilage building blocks to the body and to provide relief to sufferers of osteoarthritis pain. Some studies have shown that glucosamine supplements are effective to provide pain relief to those with osteoarthritis and are helpful to prevent cartilage damage.

Presently, most glucosamine is derived from the exoskeletons of shellfish or other marine organisms using various techniques. Although acceptable for some applications, this production method has some limitations. For example, those allergic to shellfish may try to avoid shellfish-derived glucosamine just to be safe even if unsafe allergens may be effectively removed. Other problems with the use of shellfish include the unpredictable size and composition of the shellfish used to produce glucosamine, and environmental contaminants such as heavy metals that may be concentrated in shellfish and retained in the glucosamine. Shellfish harvests are also seasonal and thus the price and availability of such may vary significantly over time. Finally, the harvest of shellfish from the world's seas and oceans may contribute to depletion of the world's marine resources. Some glucosamine users may consciously choose not to contribute to such depletion.

Another more recent method for producing glucosamine includes deriving glucosamine from microbial biomass, and more particularly from fermented fungal biomass containing chitin. These fungal biomass materials may include, for example, Aspergillus, Penicillium, or Mucor fungal species. The method generally includes reacting the fungal biomass in an acidic solution, and in particular reacting the fungal biomass in an acidic solution at elevated temperatures for a prescribed period of time. Nevertheless, this method generally includes reacting the fungal biomass for an extended period of time, typically at least four hours and in many cases more than 8 hours. These long reaction periods increase costs and slow down production rates significantly. Furthermore, the resulting glucosamine may have unacceptably high ash content in many cases.

In view of the foregoing, what is needed is a faster and more efficient method for producing safe, consistent, high-quality glucosamine from microbial biomass. Such a method would significantly reduce costs and speed up the production process. Ideally, the glucosamine produced by such a method would have a reduced ash content and could be created with minimal environmental impact.

The present invention has been developed in response to the present state of the art, and in particular, in response to the problems and needs in the art that have not yet been fully solved by currently available methods for producing glucosamine. Accordingly, the present invention has been developed to provide a more efficient method for producing glucosamine from fungal biomass. In one embodiment, a method for producing glucosamine from fungal biomass includes providing fungal biomass containing chitin, such as Aspergillus, Penicillium, Mucor, or mushroom. This fungal biomass is then reacted in an acidic solution with an acid concentration of greater than about 20% by weight and at a reaction temperature greater than 60° C in order to efficiently convert the chitin to glucosamine. This reaction is performed for a period of less than 4 hours. The glucosamine is then separated from the acidic solution.

In selected embodiments of the present invention, the method may include providing dried fungal biomass for inclusion in the reaction. This dried fungal bass may, in some embodiments, have moisture content of less than about 20%. In other embodiments, the dried fungal biomass may have less than about 13% moisture content.

In certain embodiments of the present invention, glucosamine may be separated from the acidic solution by precipitating the glucosamine from the acidic solution using a vacuum evaporator or using another suitable device or technique. Similarly, this separation step may include re-dissolving the glucosamine into solution and then re-precipitating the glucosamine from the solution to provide re-crystallized glucosamine. This step may be helpful to reduce ash content and heavy metals in the glucosamine to very low levels. In certain embodiments, the glucosamine produced by this method is at least about 98% pure based upon dry weight. In other embodiments, the purity of the glucosamine may equal or exceed about 99.9% based upon dry weight and may contain less than about 0.1 % ash.

In another embodiment of the present invention, a method for producing glucosamine from fungal biomass includes providing dried fungal biomass containing less than 20% moisture content. This dried fungal biomass is then reacted in an acidic solution with an acid concentration of greater than about 20% by weight at a reaction temperature greater than about 60° C in order to efficiently convert the chitin to glucosamine. This reaction continues for a period of less than 4 hours. The glucosamine is then separated from the acidic solution.

In yet another embodiment, a glucosamine product in accordance with the present invention is produced by the steps of providing fungal biomass containing chitin. The fungal biomass is then reacted in an acidic solution with an acid concentration of greater than about 20% by weight at a reaction temperature greater than about 60° C to convert the chitin to glucosamine. This reaction is continued for a period of less than 4 hours. The glucosamine is then separated from the acidic solution in any suitable manner.

The present invention provides novel apparatus and methods for efficiently producing glucosamine from fungal biomass. The features and advantages of the present invention will become more fully apparent from the following description and appended claims, or may be learned by the practice of the invention as set forth hereinafter.

A more particular description of the invention, as briefly described above, will be rendered by reference to specific embodiments illustrated in the appended drawings. Understanding that these drawings depict only typical embodiments of the invention and are not therefore to be considered limiting of its scope, the invention will be described and explained with additional specificity and detail through the use of the accompanying drawings, in which:
Figure 1 is a flow diagram illustrating one embodiment of a prior art method for producing glucosamine from shellfish;
Figure 2 is a flow diagram illustrating one embodiment of a prior art method for producing glucosamine from fungal biomass;
Figure 3 is a flow diagram illustrating one embodiment of a more efficient method for producing glucosamine from fungal biomass;
Figure 4 is a graph showing the yield of glucosamine over time and at different temperatures using a method in accordance with the invention;
Figure 5A is a chromatogram of a glucosamine product produced using a method in accordance with the invention; and
Figure 5B is a chromatogram of a glucosamine product produced using a method in accordance with the invention.

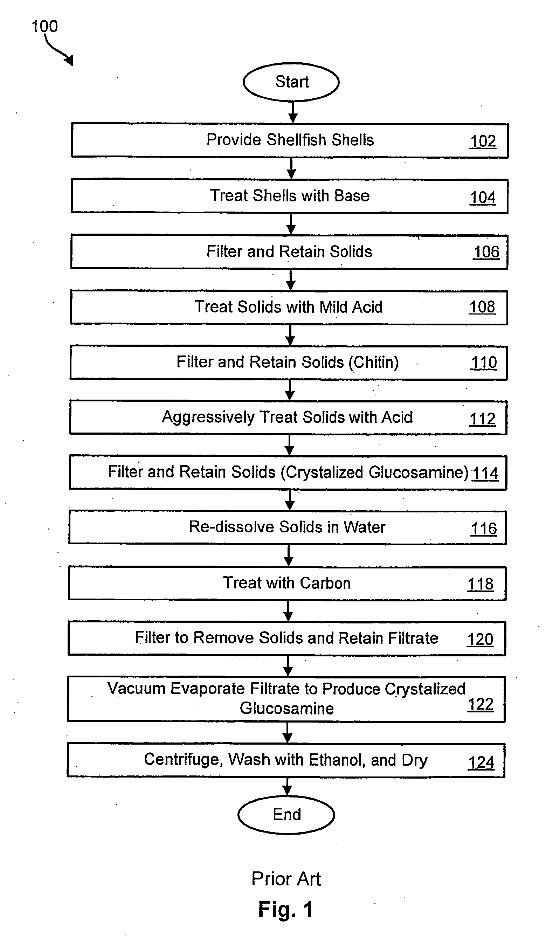

It will be readily understood that the components and process steps of the present invention, as generally described and illustrated in the Figures herein, could be arranged and designed in a wide variety of different configurations. Thus, the following more detailed description of the embodiments of methods in accordance with the present invention, as represented in Figures 1-5B, is not intended to limit the scope of the invention, as claimed, but is merely representative of certain examples of presently contemplated embodiments in accordance with the invention. The presently described embodiments will be best understood by reference to the drawings, wherein like parts are designated by like numerals throughout.

Referring to Figure 1, as mentioned, most glucosamine is currently derived from the exoskeletons of shellfish or other marine organisms, which may be obtained as byproducts of the crab, shrimp, or lobster industries. One example of a prior art method 100 for producing glucosamine from shellfish may include providing 102 shellfish shells and treating 104 the shells with a base, such as sodium hydroxide, to solubilize proteins and lipids in the shells. This solution may then be filtered 106 to remove the proteins, lipids, and other wastes, while retaining 106 the remaining solids. These solids may then be treated 108 with a mild acid to solubilize minerals and some heavy metals in the solids. The solution may then again be filtered 110 to remove the minerals and heavy metals while retaining 110 the solids.

These solids may then be subjected to an aggressive acid treatment 112 and heated over a period of time to react the chitin in the solids to obtain glucosamine. The glucosamine may then be precipitated out of the reaction slurry by cooling, adding ethanol to the slurry, or evaporating the liquid until the glucosamine reaches its solubility limit. The remaining mixture may then be filtered 114 and the filtrate discarded to remove soluble impurities as well as some soluble glucosamine in the filtrate.

The remaining solids (*i.e.,* crystallized glucosamine) may then be re-dissolved 116 in water and treated 118 with a decoloring agent such as carbon (*e.g.,* charcoal) to remove undesirable color and odor from the glucosamine. This solution may then be filtered 120 and the solids (*e.g.,* insoluble waste products, charcoal, etc.) may be discarded. The remaining filtrate may then be vacuum evaporated 122 to concentrate and precipitate out the remaining glucosamine solids. The resulting glucosamine may then be centrifuged 124, washed 124 with ethanol to remove impurities, and dried 124.

As mentioned previously, one problem with shellfish-derived glucosamine is that a significant percentage of individuals have shellfish allergies and thus avoid using products containing ingredients derived from shellfish. A large percentage of shellfish allergens are proteins which may be found in trace amounts in glucosamine derived from shellfish. Furthermore, it may not be economially feasible or even possible to remove all traces of shellfish allergens from glucosamine products. Thus, the safest path for many individuals with shellfish allergies may be to avoid the ingestion of shellfish-derived glucosamine altogether.

Another problem associated with shellfish-derived glucosamine is that it is not considered Kosher (*i.e.,* complying with Jewish dietary laws). Thus, many practicing Jews or individuals complying with Jewish dietary laws may avoid ingesting shellfish-derived products. Likewise, many vegans (*i.e.,* strict vegetarians) may refuse to ingest shellfish-derived glucosamine because it is considered an animal product. Thus, a glucosamine source is needed to satisfy individuals such as vegans or individuals complying with Jewish dietary laws.

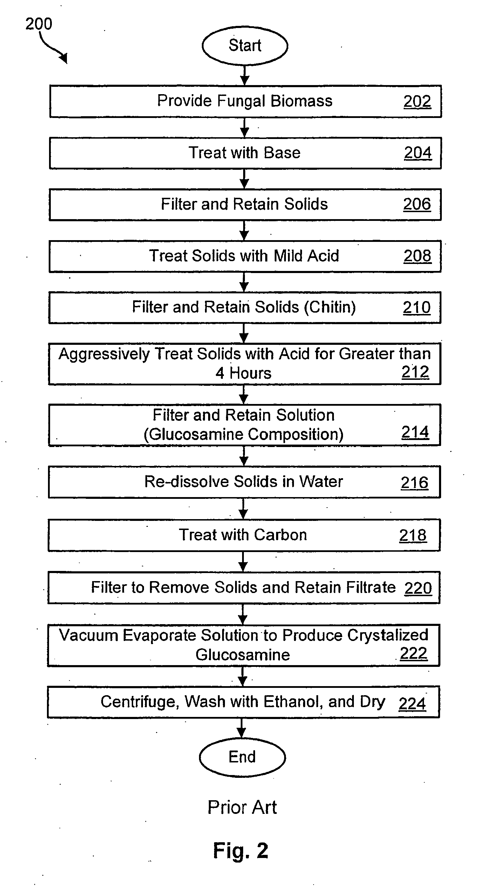

Referring to Figure 2, an alternative method for producing glucosamine that may also be considered Kosher includes producing glucosamine from microbial biomass such as fermented fungal biomass. One example of a prior art method 200 using this technique includes providing 202 fungal biomass and treating 204 it with a base (e.g., sodium hydroxide) to solubilize proteins and lipids in the biomass. This slurry may then be filtered 206 to remove the proteins and lipids while retaining 206 the solids. The solids may then be treated 208 with a mild acid (e.g., between about 1% and about 2% HCI) to break down the cell walls of the biomass without converting the chitin contained therein to glucosamine. This slurry may again be filtered 210 to remove wastes (e.g., glucans) while retaining 210 the biomass solids.

The remaining solids may then be aggressively treated 212 with acid (e.g., about 20% HCl) at elevated temperatures (*e.g.,* between about 80° C and about 106° C) and maintained under reflux conditions for a prescribed period of time (e.g., between about 4 hours to about 25 hours). This step converts the chitin to glucosamine and may also convert remaining glucans to malanoidins and levulinic acid. At this point, the slurry may be filtered 214 to remove solids (e.g., impurities) in the slurry, which may be discarded. The remaining filtrate may then be cooled which causes crystallized glucosamine to precipitate out of the solution. Alternatively, ethanol may be added to the filtrate or it may be vacuum evaporated until the glucosamine reaches its solubility limit to cause the glucosamine to precipitate out. The resulting glucosamine composition may then be filtered. The crystallized glucosamine may be retained and the filtrate, which may contain acid, water, soluble impurities, as well as some soluble glucosamine, may be discarded or recycled.

The crystallized glucosamine may optionally be subjected to further purification processing. For example, the crystallized glucosamine may be re-dissolved 216 in water and treated 218 with agents such as carbon (e.g., charcoal) to remove undesirable color and odor. This solution may then be filtered 220 and the solids (e.g., insoluble waste products, charcoal, etc.) may be discarded. The remaining filtrate may be vacuum evaporated 222 to concentrate and precipitate out the remaining glucosamine solids. This glucosamine may then be centrifuged 224, washed 224 with ethanol to remove impurities, and dried 224.

Although glucosamine derived from fungal biomass may preserve shellfish resources, be considered Kosher, and protect those with shellfish allergies, prior art methods for producing glucosamine, including the method illustrated in Figure 2, are slow and inefficient. For example, prior art methods for producing glucosamine generally include reacting the fungal biomass for an extended period of time, typically for at least four hours and in many cases more than 8 hours, in order to convert the chitin to glucosamine with satisfactory yields (*e.g*., glucosamine yields of about 50% or more based on the molar concentration of chitin in the fungal biomass).

As appreciated, these long reaction periods may increase costs and production times significantly. In some cases, the resulting glucosamine may also have unacceptably high ash content.

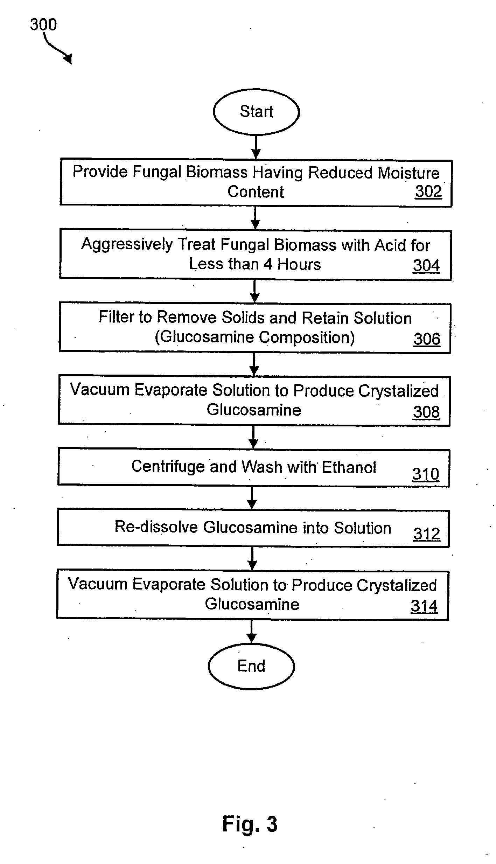

Referring to Figure 3, one embodiment of a more efficient method 300 for producing glucosamine from fungal biomass of the present invention is illustrated. Methods for producing glucosamine in accordance with the invention, like that illustrated in Figure 3, exhibit various advantages over prior art processes, including but not limited to fewer processing steps, shortened reaction time, lower ash content, and the like. One skilled in the art, however, will recognize that the illustrated method 300 may be practiced without one or more of the illustrated steps, or with additional or alternative steps, methods, or techniques to achieve the same or substantially same result. In some cases, well-known steps or techniques may not be shown or described in detail to avoid obscuring aspects of the invention. Thus, the illustrated method 300 is not provided with the intent to limit the scope of the invention to the illustrated steps, but is merely representative of one presently contemplated embodiment of a method in accordance with the invention.

In certain embodiments, a method 300 in accordance with the invention may include providing 302 fungal biomass having reduced moisture content, such as dried fungal biomass. In certain embodiments, suitable starting materials may include filamentous fungal biomass having significant chitin content. For example, it has been shown that suitable starting materials may include fungal biomass derived from various species of Aspergillus, Penicillium, and Mucor molds. This fungal biomass (*e.g*., citric biomass) may often be obtained from commercial fermentation processes, such as from commercial production of organic acids. Nevertheless, the biomass may also be produced specifically for glucosamine production as opposed to being a byproduct of other processes.

Mushroom may also provide a suitable starting material for a process 300. For example, Agaricus bisporus is the most commonly produced mushroom in the United States. The commercial production of this mushroom produces a large amount of waste in the form of stalks, irregularly shaped mushrooms, and compost. These wastes are rich in chitin and thus may provide a good starting material for generating glucosamine using the method 300 described herein.

The fungal biomass may also be provided with reduced moisture content. In certain embodiments, the moisture content may be less than about 20% and in some cases less than about 13% of the biomass weight. This may be accomplished by drying or otherwise removing water from the biomass. In certain embodiments as contemplated by the present invention, the water may be removed by evaporation, which may include air drying, sun drying, wind drying, or the like. This process may be accelerated by placing the fungal biomass in an oven, near a heat source, or by compressing the biomass to remove water. In other contemplated embodiments, water may be removed by freezing the biomass and then reducing the surrounding pressure to allow the frozen water to dissipate through sublimation (*i.e.,* freeze-drying).

The reduced moisture content of the fungal biomass is useful to accelerate the glucosamine production process by increasing the acid concentration and thus reducing the time needed to convert the chitin to glucosamine. This step also differs significantly from prior art processes, which generally use fungal biomass having greater than about 50% moisture content. Furthermore, the novel method of the present invention 300 may eliminate the need to pre-treat the fungal biomass with an alkali, as is taught by some prior art processes.

The drier fungal biomass may be aggressively treated 304 with acid for a period of less than 4 hours. In certain embodiments, the biomass may be treated 304 between about 1 hour and about 4 hours, and in others between about 2 hours and about 3 hours in order to provide the desired yield of glucosamine. Strong acids (*e.g*., hydrochloric, phosphoric, sulfuric, citric acids, etc.) of concentrations of less than about 60%, and in some cases between about 20% and about 40% may be used to hydrolyze the fungal biomass. Acid hydrolysis may be used to break linkages in the biomass and deacetylate the chitin molecules to generate substantially intact glucosamine molecules. Acid hydrolysis may also be used to break down components such as glucans, proteins, and lipids in the fungal biomass.

In selected embodiments, the glucosamine-producing reaction may be conducted with between about 20% and about 30 % dry biomass, between about 15% and about 30% acid, and between about 40% and about 65% water by weight. In other embodiments, the reaction slurry may include between about 25% and about 30% dry biomass and about 70% and about 75% hydrochloric acid, wherein the concentration of the acid is between about 25% and about 32%.

This slurry containing the biomass, acid, and water, if any, may be heated to an elevated temperature and maintained at or near this temperature for a prescribed period of time. For example, the slurry may be heated to a temperature above about 60° C and more commonly between about 80° C and about 110° C and maintained at reflux for less than 4 hours, more typically between about 2 hours and about 3 hours. The reaction time should be maintained long enough to maximize the breakdown of chitin, but kept sufficiently short to minimize the breakdown of glucosamine.

At this point, the slurry may be filtered 306 to remove solids (*e.g.,* impurities), which may be discarded. The remaining solution may then be cooled, causing a glucosamine composition to precipitate out of the solution. The glucosamine may also be precipitated out of the solution by adding ethanol or by evaporating 308 the solution until the glucosamine composition reaches its solubility limit. The solid glucosamine composition may then be retained and the filtrate, which may contain acid, water, soluble impurities, as well as some soluble glucosamine, may be discarded. The glucosamine may then be recovered from the glucosamine composition by filtration or centrifugation 310, after which it may be washed 310 with ethanol and dried 310.

The glucosamine may than be subjected to further purification processing, if desired. For example, the crystallized glucosamine may be re-dissolved 312 in water or another solvent. This solution may then be filtered and the solids (*e.g.,* insoluble waste products) may be discarded. The remaining filtrate may then be vacuum evaporated 314 to concentrate and precipitate out the glucosamine. The purity of this re-crystallized glucosamine has been found to exceed about 98% with ash content of less than about 2% and, more typically, the glucosamine exceeds 99.9% purity with ash content of less than 0.1%. Similarly, the heavy metal content in the re-crystallized glucosamine is typically very low, usually significantly below about 50 parts per million, more typically below about 20 parts per million, and even more typically below about 10 parts per million. Glucosamine produced by a method in accordance with the present invention has been found to produce glucosamine yields of about 55% or more based on the molar concentration of chitin in the fungal biomass.

The following are several non-limiting examples of methods used to produce glucosamine from fungal biomass. Unless otherwise indicated, all amounts are expressed in parts by weight.

### EXAMPLE I

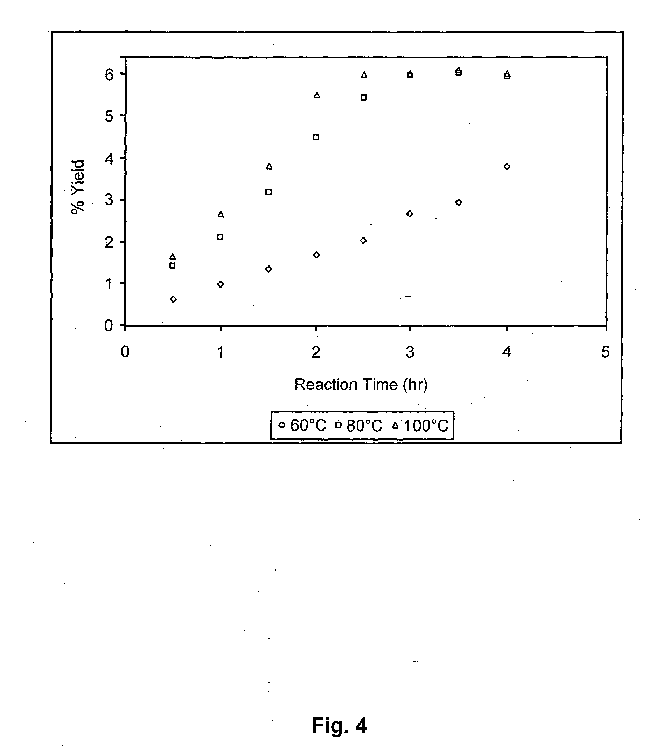

Dried citric biomass in the amount of 100 grams was mixed with 250 milliliters of 20% hydrochloric acid (HCI). This mixture was heated at reflux in three experiments at three different temperatures: 60° C, 80° C, and 100° C. Samples were taken at various intervals, and the reaction was analyzed with a high-pressure liquid chromatograph available from Agilent HPLC under the trade name Agilent 1100. The results of this reaction are provided in Figure 4, which provides a graph showing one example of glucosamine production as a function of reaction time and temperature. As shown, the initial 100 grams of dried biomass, which contains approximately 10 grams of chitin, yields approximately 6 grams of glucosamine. This provides a 6% yield relative to the initial 100 grams of dried biomass, or a 60% yield relative to the 10 grams of chitin contained in the dried biomass.

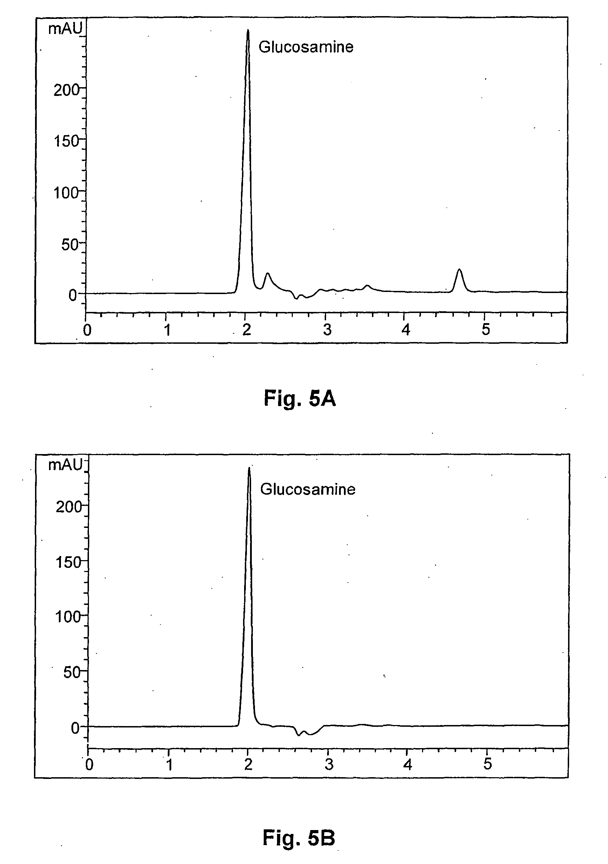

As can be seen from the graph, the reactions conducted at 80° C and 100° C produced a greater yield of glucosamine than the reaction conducted at 60° C. As is also evident, the yield of glucosamine increased and then leveled off at approximately 3 hours for the reactions conducted at 80° C and 100° C. Thus, the glucosamine yield did not increase significantly after 3 hours. As can also be seen, the glucosamine yield was substantially equal after 3 hours for the reactions conducted at 80° C and 100° C. A chromatogram of the glucosamine sample taken at 2 hours at hydrolysis conditions of 100° C is shown in Figure 5A, which shows the glucosamine has a purity of approximately 71.3% at this stage in the process. This purity will continue to increase as the reaction continues.

Following the reaction, the slurry was filtered and the filtrate evaporated using a rotating evaporator manufactured by Senco to increase the glucosamine concentration in the solution. The final volume was reduced to between about 15 milliliters and about 30 milliliters. About 50 milliliters of ethanol was then added to this solution and the mixture swirled to promote precipitation of the glucosamine and enhance yield. The glucosamine precipitates were then filtered from the solution and washed with alcohol until the color became substantially white. The precipitated glucosamine was then dissolved in water and then re-crystallized using the evaporation techniques described above.

Figure 5B shows a chromatogram of the final product, indicating a purity of greater than 98% glucosamine.

### EXAMPLE II

Dried citric biomass in the amount of 100 grams was mixed with 300 milliliters of 31% HCl. This mixture was then heated at reflux for 2.5 hours at 100° C. Following the reaction, the mixture was filtered and the filtrate was evaporated to between about 15 milliliters and about 30 milliliters. About 50 milliliters of ethanol was then added to this solution and the mixture swirled to promote precipitation of the glucosamine. The glucosamine precipitates were then filtered from the solution and washed with alcohol. The precipitated glucosamine was then dissolved in water and re-crystallized using the evaporator. The final product provided glucosamine with greater than 98% purity.

### EXAMPLE III

Dried mushroom in the amount of 100 grams was mixed with 300 milliliters of 31% HCl. This mixture was then heated at reflux for 2.5 hours at 100° C. Following the reaction, the mixture was filtered and the filtrate was evaporated between about 15 milliliters and about 30 milliliters. About 50 milliliters of ethanol was then added to this solution and the mixture swirled to promote precipitation of the glucosamine. The glucosamine precipitates were then filtered from the solution and washed with alcohol. The precipitated glucosamine was then dissolved in water and re-crystallized using the evaporator. The final product provided glucosamine with greater than 98% purity.

The present invention may be embodied in other specific forms without departing from its spirit or essential characteristics. The described embodiments and examples are therefore to be considered in all respects only as illustrative and not restrictive. The scope of the invention is, accordingly, indicated by the appended claims rather than by the foregoing description. All changes which come within the meaning and range of equivalency of the claims are to be embraced within their scope.

## Claims

1. A method for producing glucosamine from microbial biomass, said method comprising the steps of:
providing microbial biomass containing chitin;
reacting said microbial biomass in an acidic solution with an acid concentration of greater than about 20% by weight at a reaction temperature greater than about 60° C for a reaction period of less than 4 hours to convert said chitin to glucosamine; and
separating said glucosamine from said acidic solution.

2. A method as claimed in claim 1, wherein said step of providing microbial biomass comprises providing dried fungal biomass.

3. A method as claimed in claim 1 or 2, wherein said dried fungal biomass comprises less than 20% moisture content.

4. A method as claimed in any preceding claim, wherein said dried fungal biomass comprises less than 13% moisture content.

5. A method as claimed in any preceding claim, wherein said step of separating said glucosamine comprises the step of precipitating the glucosamine from said acidic solution.

6. A method as claimed in claim 5, further comprises the step of re-dissolving said precipitated glucosamine into said acidic solution.

7. A method as claimed in claim 6, wherein separating said glucosamine further comprises the step of re-precipitating the glucosamine from said acidic solution.

8. A method as claimed in claim 7, wherein said resulting glucosamine is at least about 98% pure based upon dry weight.

9. A method as claimed in claim 8, wherein said resulting glucosamine is at least about 99.9% pure based upon dry weight.

10. A method as claimed in any preceding claim, wherein said reaction period is between about 1 hour and about 4 hours.

11. A method as claimed in claim 10, wherein said reaction period is between about 2 hours and about 3 hours.

12. A method as claimed in any preceding claim, wherein said acid concentration is between about 20% and about 60% by weight.

13. A method as claimed in any preceding claim, wherein said glucosamine has a yield of at least about 50% based on a molar concentration of said chitin in said fungal biomass.

14. A method as claimed in any of claims 2 to 13, wherein said fungal biomass is selected from the group consisting of Aspergillus, Penicillium, Mucor, and mushroom.

15. A method for obtaining glucosamine from fungal biomass, said method comprising the steps of:
providing dried fungal biomass containing less than about 20% moisture content;
reacting said fungal biomass in an acidic solution at a reaction temperature greater than about 60° C for a reaction period of less than 4 hours to convert said chitin to glucosamine; and
separating said glucosamine from said acidic solution.

16. A method as claimed in claim 15, wherein said dried fungal biomass comprises less than about 13% moisture content.

17. A method as claimed in claim 15 or 16, wherein said step of separating said glucosamine further comprises the step of precipitating the glucosamine from said acidic solution, re-dissolving the glucosamine into said acidic solution, and re-precipitating the glucosamine from the acidic solution.

18. A method as claimed in any of claims 15 to 17, wherein said glucosamine comprises an ash content of less than about 0.1 %.

19. A method as claimed in any of claims 15 to 17, wherein said resulting glucosamine is at least about 99.9% pure based upon dry weight.

20. A method as claimed in any of claims 15 to 19, wherein said reaction period is between about 1 hour and about 4 hours.

21. A glucosamine product obtained or obtainable from the method of any preceding claim.

22. A glucosamine product as claimed in claim 21 which is at least about 98% pure based upon dry weight.

23. A glucosamine product as claimed in claim 21 or 22 which is at least about 99.9% pure based upon dry weight
